Europäisches Patentamt

European Patent Office

Office européen des brevets

(19)

(11) Publication number : 0 503 741 A1

(12) **EUROPEAN PATENT APPLICATION**

(21) Application number : 92200700.0

(51) Int. Cl.⁵ : **A61K 47/48**

(22) Date of filing : 11.03.92

PRIORITY 12.03.91 IT MI91000656.

(30) Priority : **12.03.91**

(43) Date of publication of application :
**16.09.92 Bulletin 92/38**

(84) Designated Contracting States :
**DE ES FR GB PT**

(71) Applicant : **LABORATORI BALDACCI Spa**
**Via San Michele degli Scalzi, 73**
**I-56100 Pisa (IT)**

(72) Inventor : **Fiume, Luigi**
**Via Dante, 7**
**I-40125 Bologna (IT)**
Inventor : **Busi, Corrado**
**Via Berretta Rossa, 68**
**I-40133 Bologna (IT)**
Inventor : **Mattioli, Alessandro**
**Via Ravenna, 14**
**I-40139 Bologna (IT)**
Inventor : **Baldacci, Massimo**
**Via delle Piagge, 9**
**I-56100 Pisa (IT)**

(74) Representative : **Michelotti, Giuliano et al**
**c/o SAIC BREVETTI S.r.l. Viale Bianca Maria**
**15**
**I-20122 Milano (IT)**

(54) **Conjugate of azidothymidine and human albumin, process for its preparation and pharmaceutical compositions containing it.**

(57)    The conjugate of azidothymidine with human albumin is useful in the therapy for inhibiting the replication of the HIV virus which is responsible of AIDS, selectively entering the cells of RES which are specifically seat of the viral infection.
   For the preparation of the conjugate a derivative of azidothymidine, preferably the monophosphate, and human albumin, are reacted in aqueous solution, by adjusting the pH to a value from neutral to weakly alkaline and adding to the reaction mixture a condensating agent, preferably 1-ethyl-3(dimethyl-aminopropyl)carbodiimide, the conjugate being obtained after incubation of the reaction mixture at a room temperature and for a time sufficient for the forming of the conjugate.
   The pharmaceutical compositions useful for the indicated therapeutical purpose are provided with the active ingredient, namely the conjugate product, in lyophilized form.

EP 0 503 741 A1

The present invention relates to a novel compound useful in the therapy of AIDS and more specifically a novel conjugate of azidothymidine (AZT).

It is known that azidothymidine is effective in the inhibition of the replication of the HIV virus which is responsible of AIDS; this drug, however, is subjected to heavy use limitations, since it induces toxic effects in the bone marrow.

It is also known that some antiviral drugs, after coniugation with human albumin, selectively enter, both in vitro and in vivo, the cells of the reticulo endothelial system (RES) in which are released in pharmacologically active form (Balboni P.G. at al., Nature 264, 181, ( 1976)).

Since the infection of macrophagi belonging to the RES (Furth R.van at all, Bull. Wld. Org., 46,845 81972), induced by HIV seems to play a particularly important role in the AIDS development (Fauci A.S., Science, 239, 617 (1988)), it is evident that it would be highly desirable to be able to selectively address the active drug into the cells which are specifically seat of the viral infection.

It has been now found and is the main object of the present invention that the conjugate of azidothymidine with human albumin has the property of selectively enter the macrophagi of the reticulo endothelial system releasing therein the AZT.

Another object of the present invention is the process for the preparation of the above defined conjugate, which process consists in reacting human albumin in form of aqueous solution with the monophosphate derivative of azidothymidine, prepared according to the known art (Yoshikawa M. et al., Tetrahedron Lett., 50,5065 (1967)), also in aqueous solution, by adjusting the pH of the resulting reaction mixture to a value from neutral to weakly alcaline and adding 1-ethyl-3-(dimethyl-aminopropyl)carbodiimide, the reaction mixture being maintained in incubation at room temperature and under stirring until the desired conjugate is formed and then isolated.

In the practice of the process of according to the present invention equal weight amounts of human albumin and monophosphate derivative of AZT are dissolved in water and in the resulting aqueous solution is added with 10N NaOH, until the pH attains the desired value, preferably 7.5.

After the addition of carbodiimide the resulting mixture is maintained in incubation for 24 hours at 25°C under stirring; upon this time is lapsed the resulting conjugate is cold dialyzed against 0.9% NaCl, then effecting the standard concentration and lyophilization operations.

From the analyses carried on the resulting conjugate it has been found that the molar ratio AZT/protein, determined by calculating the concentration of AZT through the estinction at 268 nm ($E^1\%=377\,800$) after subtraction of the absorption of albumin at the same wave length ($E^1\%=4450$), is of between 8 and 10. The albumin concentration is determined according to the method of Lowry et al. (Lowry O.H. et al., J.Biol.Chem., 193, 265 (1951)).

The solubility of the conjugate remains constant in the long run. As a matter of fact by maintaining the lyophilized conjugate at room temperature it remains soluble at 10 mg/ml up to six months after the preparation (the latter having no meaning as a time limit, since it is only the maximum storage period experimented to date).

The SDS electrophoresis of the conjugate on polyacrylamide gel reveals that it consists of the monomer and of oligomers of HSA which are formed during the conjugation reaction in the presence of carbodiimide. The percent composition as monomer, dimer, trimer and other higher oligomers as determined on a conjugate prepared as disclosed in the following example, as been found as follows: 52,23,9, 16.

Hereinafter the acid insoluble radioactivity is reported, per mg of cellular protein, as measured in the sinusoidal cells (which are part of the Reticulo Endothelial System) and in the parenchyma cells of the rat liver after administration of the conjugate marked in the proteic moiety. The results indicate the amount of the conjugate which penetrate into different types of cells.

From the table it is noted that the radioactivity in the sinusoidal cells is 20-30 times higher than that in the parenchymal cells thus confirming the selective penetration of the conjugate in the RES cells.

Acid insoluble radioactivity

dpm x $10^4$/mg cellular protein

specific activity of conjugate

| Conjugate | Dose | Interval (a) | Epatocytes | Synusoidal cells |
|---|---|---|---|---|
| $^3$H HSA–AZTMP | ( μg/g) | (min) | | |
| | | 7 | 99± 10 | 2633 ± 83 |

Each result represents the average (± standard error) of the values obtained from 3 animals.

a) Interval between the administration of the conjugate and the beginning of the liver perfusion with collagenase to separate hepatic cells.

These cells have been isolated by using the proceeding described by Segle (Methods Cell. Biol. 13 29 ( 1976)).

An example of preparation of the conjugate according to the process of the present invention is now provided, obviously having only illustrative and in any way non limitative title.

The conjugation of AZT with human albumin has been preceeded by the conversion of the drug into its monophosphate derivative (AZTMP) according to the method of Yoshikawa et al., (4). The phosphorylation has been effected by repeatedly adding, to a suspension of AZT (1 g) in triethylphosphate (10 ml) maintained at temperature of between 0 and 3°C, small amounts of phosphor oxycloride (200 μl) until the drug is completely phosphorylated.

The behavour of the phosphorylation reaction has been monitored by thin layer chromatography using plates coated with silica gel and, as eluant, a mixture comprising isopropanol: ammonia: water (52:27:20).

The AZTMP has been purified by absorption on a column of activated carbon (Bio Rad, 1.6 x 30 cm). The column has been washed with water up to full elimination of chlorine ions (AgNo$_3$ test) and until no other reading was found in the washing liquid at 268 nm. The AZTMP, retained from the column, has been eluted with ethyl alcool:water:ammonia (70:24:6). The eluant has been removed by evaporation under vacuum and the residue, taken with H$_2$O, has been lyophilized. Inorganic phosphate was absent in the product (5) and the ratio base: organic phosphate (5) was practically 1: 1.

The HPLC of the product (on a column μBondpack C$_{ie}$ eluted as described by McCann et al., (6)) shows a peak forming 97-98% of the chromatogram; a second very small peak (2-3%) has the same retention time as AZT.

The product, after dephosforilation carried out by using alcaline phosphatase of Escherichia coli, upon being analized by HPLC, shows only one peak having the same retention time of an authentic sample of AZT.

The AZTMP-HSA conjugate has been prepared by dissolving in 6 ml of H$_2$O 300 mg of AZTMP and 300 mg of HSA. The pH of the solution has been brought to 7.5 by 10N NaOH and then 300 mg of 1-ethyl-3-(dimethyl-aminopropil)carboiimide have been added. After 24 hours incubation at 25°C under stirring the conjugate has been cold dialyzed again 0.9% NaCl. Then it has been concentrated to 100 mg/ml on Minicon B15 cells(Amicon) and divided in 1 ml fractions (each one contain 100 mg conjugate and 9 mg of NaCl) which have been lyophilized. When requested, each fraction has been dissolved in 1 ml of H$_2$O and then, if necessary, diluted with 0.9% NaCl.

As regards the therapeutical use of the conjugate derivative of AZT according to the present invention, the administration is foreseen of doses corresponding to the same amounts of AZT used to date, with the evident advantage of concentrating the action of the drug on the cells on which it has to act.

In the above description specific reference has been made to the conjugation of human albumin with AZT monophosphate, it being meant that the same preparation is also possible and foreseable with other derivatives of azidothymidine.

## Claims

1. Conjugate of azidothymidine with human albumin.

2. Conjugate according to claim 1, characterized in that the molar ratio between azidotymidine and albumin is of between 8 and 10.

3. A process for the preparation of the conjugate according to claim 1, characterized in that human albumin and a water soluble derivative of azidothymidine, both in aqueous solvent, are reacted, by adjusting the pH of the reaction mixture to a value from neutral to weakly acidic, and adding to the reaction mixture a condensating agent, the reaction mixture being maintained in incubation until the desired conjugate is formed.

4. A process according to claim 3, characterized in that said derivative of azidothymidine is monophosphate.

5. A process according to claim 3, characterized in that the reaction mixture is prepared by dissolving in water the derivative of azidothymidine and the human albumin, adjusting the pH of the reaction mixture to 7.5 through the addition of a strong base, preferably NaOH.

6. A process according to claim 3, characterized in that the said condensation agent is 1-ethyl-3(dimethyl-amminopropil)carbodiimide.

7. Process according to calim 3, characterized in that said incubation phase is effected at room temperature and for 24 hours.

8. Pharmaceutical composition characterized by containing as active ingredient the conjugate according to claim 1.

9. Pharmaceutical composition according to claim 8, characterized in that said active ingredient is in lyophilized form.

**European Patent Office**

# EUROPEAN SEARCH REPORT

Application Number

EP 92 20 0700

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | FR-A-2 637 897 (SIGMA CHEMICAL CORPORATION) | 1-9 | A61K47/48 |
| Y | * claims 1-10; examples 39,40 * | 1-9 | |
| X | WORLD PATENTS INDEX LATEST<br>Section Ch, Week 9015,<br>Derwent Publications Ltd., London, GB;<br>Class B, AN 90-110454<br>& JP-A-2 059 526 (TOSOH CORP) 28 February 1990 | 1,2,8,9 | |
| Y | * abstract * | 1-9 | |
| Y | EP-A-0 220 657 (LABORATORI BALDACCI SPA)<br>* claims 1-7 * | 1-9 | |
| Y | EP-A-0 184 838 (LABORATORI BALDACCI SPA)<br>* the whole document * | 1-9 | |
| Y | BIOCHEMICAL PHARMACOLOGY<br>vol. 40, no. 12, 1990, OXFORD,G.B.<br>pages 2603 - 2610;<br>MOLEMA ET AL: 'TARGETTING OF ANTIVIRAL DRUGS TO<br>T4-LYMPHOCYTES;ANTI-HIV ACTIVITY OF<br>NEOGLYCOPROTEIN-AZTMP CONJUGATES IN VITRO'<br>* the whole document * | 1-9 | TECHNICAL FIELDS SEARCHED (Int. Cl.5)<br><br>A61K |
| Y | JOURNAL OF MEDICINAL CHEMISTRY<br>vol. 34, 1991, WASHINGTON D.C.,USA<br>pages 1137 - 1141;<br>MOLEMA ET AL: 'NEOGLYCOPROTEINS AS CARRIERS FOR<br>ANTIVIRAL DRUGS:SYNTHESIS AND ANALYSIS OF<br>PROTEIN-DRUG CONJUGATES'<br>* the whole document * | 1-9 | |

The present search report has been drawn up for all claims

| Place of search | Date of completion of the search | Examiner |
|---|---|---|
| THE HAGUE | 10 JUNE 1992 | SITCH W.D.C. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document